# EUROPÄISCHE PATENTANMELDUNG

(11) **EP 4 748 456 A1**
(43) Veröffentlichungstag der Anmeldung: **27.05.2026**
(21) Anmeldenummer: 25213167.7
(22) Anmeldetag: 04.11.2025
(51) Int. Cl.: A62B 7/02, A61B 5/08, A61B 5/091, A61B 5/22, A62B 99/00, A62C 99/00, A63B 22/00

(54) **ÜBUNGSANLAGE FÜR ANWENDER EINES ATEMSCHUTZGERÄTES, ÜBUNGSSYSTEM, VERFAHREN UND COMPUTERPROGRAMMPRODUKT**

(30) Priorität: 15.11.2024 DE 102024133423
(71) Anmelder: Dräger Safety AG & Co. KGaA, 23560 Lübeck (DE)
(72) Erfinder: Nieß, Michael, 23558 Lübeck (DE); Müller, Bernd, 23558 Lübeck (DE)

(57) **Zusammenfassung**

Übungsanlage (1) für Anwender (21) eines Atemschutzgerätes (5) umfassend eine Steuereinheit (3) und wenigstens ein Übungsgerät (2a, 2b, 2c) zur physischen Belastung des Anwenders (21), wobei die Steuereinheit (3) ein Kommunikationsmodul (4) umfasst, das eingerichtet ist, einen Atemluftentnahmewert (A), der von einem Atemschutzgerät (5) ermittelbar ist, und einen Belastungswert (B1, B2, B3), der von dem wenigstens einen Übungsgerät (2a, 2b, 2c) ermittelbar ist, zu empfangen, wobei der Belastungswert (B1, B2, B3) eine Information über eine verrichtete Arbeit des Anwenders (21) an dem wenigstens einen Übungsgerät (2a, 2b, 2c) umfasst, und wobei die Steuereinheit (3) eingerichtet ist, auf Grundlage des Atemluftentnahmewertes (A) und des Belastungswertes (B1, B2, B3) einen Übungsstatus zu ermitteln und ein Ausgabesignal (S) zu erzeugen, das eine Information über den Übungsstatus umfasst. Ferner betrifft die Erfindung ein Übungssystem 20, ein Verfahren und ein Computerprogrammprodukt.

## Beschreibung

### Beschreibung

Die vorliegende Erfindung betrifft eine Übungsanlage für Anwender eines Atemschutzgerätes, ein Übungssystem umfassend ein Atemschutzgerät und eine Übungsanlage sowie ein Verfahren und ein Computerprogrammprodukt zur automatisierten Überwachung eines Übungsstatus eines Anwenders eines Atemschutzgerätes.

Atemschutzgeräte schützen den Atemschutzgeräteträger vor gesundheitsschädlichen Stoffen, die über die Atemwege in den Körper gelangen können. Im Sinne der Erfindung sind unter Atemschutzgeräten umgebungsluftunabhängige Atemschutzgeräte, Isoliergeräte, Pressluftatmer sowie auch Drucklufttauchgeräte und/oder Presslufttauchgeräte zu verstehen. Üblicherweise verfügen diese Atemschutzgeräte über einen Atemluftvorrat, wobei die Versorgung des Anwenders mit Atemluft unabhängig von der Umgebungsluft ist.

Anwender von Atemschutzgeräten sind beispielsweise Rettungskräfte der Feuerwehr, des Technischen Hilfswerkes oder des Rettungswesens. Dabei ist es wichtig, dass die Anwender in der Verwendung von Atemschutzgeräten geübt und dafür geeignet sind, wobei eine gute, körperliche Verfassung notwendig ist. Zur Sicherstellung der Eignung eines Anwenders für die Verwendung eines Atemschutzgerätes ist es üblich, die Eignung in einer Übungsanlage zu prüfen. Dabei wird geprüft, wie der Atemschutzgeräteträger auf körperliche Belastung reagiert und wie hoch sein Atemluftverbrauch dabei ist.

Eine Eignungsprüfung von Anwendern eines Atemschutzgerätes findet üblicherweise regelmäßig, beispielsweise jährlich, statt, so dass sichergestellt ist, dass der Anwender für Einsätze unter Atemschutz geeignet ist. Dabei wird in der Übungsanlage mit einem oder mehreren Übungsgeräten eine Einsatzsituation simuliert, wobei der Anwender eine physische Belastung erfährt, die seine Atmung und/oder seinen Atemluftverbrauch beeinflusst. Zur Bewertung, ob der Anwender geeignet ist, wird in der Praxis die Atemluft ermittelt, die während der Übung vom Anwender verbraucht wird. Ist die verbrauchte Menge von Atemluft bei einer festgelegten physischen Belastung kleiner als ein vorgegebener Grenzwert, so ist davon auszugehen, dass der Anwender für einen Einsatz unter Atemschutz geeignet ist. Wird dieser Grenzwert verletzt, so ist der Anwender nicht geeignet und darf an Einsätzen unter Atemschutz nicht teilnehmen. Häufig ist es notwendig, dass der Anwender in diesem Fall beispielsweise ein Training zur Konditionsverbesserung durchführen muss.

Die Ermittlung der vom Atemschutzgeräteträger verbrauchten Atemluft wird üblicherweise durch manuelles Ablesen des Atemluftvorrates vor und nach der Übung ermittelt. Dementsprechend ist für den Anwender des Atemschutzgerätes oder einen Übungsleiter während der Übung in der Übungsanlage nicht erkennbar, ob der Atemluftverbrauch in einem zulässigen Bereich ist. Ferner kann es vorkommen, dass der Atemluftvorrat nicht für die Durchführung der gesamten Übung genügt, so dass die Übung abgebrochen werden muss oder der Anwender wegen unzureichender Atemluftversorgung einem gesundheitlichen Risiko ausgesetzt ist. Insbesondere bei Übungsanlagen mit einer Mehrzahl von Übungsgeräten ist mit der bekannten Ermittlung des Atemluftverbrauchs nicht feststellbar, an welchem Übungsgerät oder bei welcher Art der physischen Belastung der Anwender besonders viel Atemluft verbraucht hat. Diese Information ist jedoch für ein gezieltes Training, beispielsweise an einem bestimmten Übungsgerät, in Vorbereitung auf eine erneute Eignungsprüfung notwendig.

Ausgehend von den aus dem Stand der Technik bekannten Lösungen sowie den zuvor geschilderten Problemen liegt der Erfindung die Aufgabe zu Grunde, eine Übungsanlage sowie ein Übungssystem für Anwender eines Atemschutzgerätes bereit zu stellen, welche eine sichere und zuverlässige Prüfung der Eignung des Anwenders für die Nutzung des Atemschutzgerätes ermöglichen. Ferner sollen ein Verfahren und ein Computerprogrammprodukt zur automatisierten Überwachung eines Übungsstatus eines Anwenders eines Atemschutzgerätes angegeben werden.

Die voranstehende Aufgabe wird durch eine Übungsanlage mit den Merkmalen des Anspruchs 1, einem Übungssystem gemäß Anspruch 6 sowie einem Verfahren gemäß Anspruch 7 und einem Computerprogrammprodukt gemäß Anspruch 8 gelöst. Weitere Details der Erfindung ergeben sich aus den abhängigen Ansprüchen, der Beschreibung und den Zeichnungen. Dabei gelten Merkmale und Details, die im Zusammenhang mit der erfindungsgemäßen Übungsanlage beschrieben sind, auch im Zusammenhang mit dem erfindungsgemäßen Übungssystem sowie dem erfindungsgemäßen Verfahren und dem erfindungsgemäßen Computerprogrammprodukt, sodass bezüglich der Offenbarung zu den einzelnen Erfindungsaspekten stets wechselseitig Bezug genommen wird oder vielmehr genommen werden kann.

Die erfindungsgemäße Übungsanlage, insbesondere eine Atemschutzübungsanlage, für Anwender eines Atemschutzgerätes umfasst wenigstens ein Übungsgerät zur physischen Belastung des Anwenders und eine Steuereinheit. Die Steuereinheit umfasst ein Kommunikationsmodul, das eingerichtet ist, einen Atemluftentnahmewert, der von einem Atemschutzgerät ermittelbar ist, zu empfangen. Bei dem vom Kommunikationsmodul empfangbaren Atemluftentnahmewert handelt es sich insbesondere um einen vom Atemschutzgerät des Anwenders ermittelten Atemluftentnahmewert. Dieser Atemluftentnahmewert wird insbesondere dann ermittelt und empfangen, wenn der Anwender das Atemschutzgerät zur Versorgung mit Atemluft benutzt und/oder mit seinem Atemschutzgerät an dem wenigstens einen Übungsgerät der Übungsanlage Arbeit verrichtet und/oder trainiert. Der Atemluftentnahmewert umfasst einen Wert für eine Atemluftmenge, beispielsweise einen Volumenwert, und/oder eine Atemluftmenge pro Zeiteinheit. Ferner ist das Kommunikationsmodul eingerichtet, einen Belastungswert, der von dem wenigstens einen Übungsgerät ermittelbar ist, zu empfangen. Dabei umfasst der Belastungswert eine Information über eine verrichtete Arbeit, insbesondere aufgrund einer physischen Belastung, des Anwenders an dem wenigstens einen Übungsgerät. Die Steuereinheit ist eingerichtet, auf Grundlage des Atemluftentnahmewertes und des Belastungswertes einen Übungsstatus zu ermitteln und ein Ausgabesignal zu erzeugen, das eine Information über den Übungsstatus umfasst.

Der Übungsstatus gibt insbesondere an, ob eine vom Anwender benötigte Atemluftmenge und eine verrichtete Arbeit während und/oder nach der Benutzung des wenigstens einen Übungsgerätes, also der physischen Belastung des Anwenders, vorbestimmten Kriterien genügt. Dabei sind die Kriterien so gewählt, dass eine Eignung des Anwenders zum Tragen eines Atemschutzgerätes feststellbar ist. Dies wird in der weiteren Beschreibung noch näher erläutert.

Unter Atemschutzgerät ist insbesondere ein umgebungsluftunabhängiges Atemschutzgerät, ein Isoliergeräte oder ein Pressluftatmer zu verstehen. Ferner ist auch ein Tauchgerät, insbesondere ein Drucklufttauchgerät oder ein Presslufttauchgerät, als Atemschutzgerät zu verstehen.

Die Übungsanlage umfasst zumindest ein Übungsgerät, an dem der Anwender Arbeit verrichten kann und dementsprechend einer physischen Belastung ausgesetzt ist, die aufgrund der Anstrengung des Anwenders Einfluss auf dessen Atemluftverbrauch hat. Das wenigstens eine Übungsgerät ist beispielsweise ein Laufband, ein Crosswalker, ein Crosstrainer, ein Armergometer, ein Schlaggerät, eine Endlosleiter, ein Fahrradergometer, ein Ruderergometer oder ein Stepper. Weitere gattungsähnliche Übungsgeräte sind denkbar. Das wenigstens eine Übungsgerät ist vorzugsweise eingerichtet, die vom Anwender an dem wenigstens einen Übungsgerät verrichtete Arbeit zu ermitteln und als Belastungswert bereit zu stellen. Bevorzugt ist der Belastungswert in Joule ermittelbar.

Ferner ist denkbar, dass das wenigstens eine Übungsgerät eine Hindernisstrecke ist. Die Hindernisstrecke umfasst dabei beispielsweise eine Parkour mit mehreren Hindernisse zwischen einem Startpunkt und einem Zielpunkt, den der Anwender überwinden muss und dabei Arbeit verrichtet.

Diese verrichtet Arbeit in der Hindernisstrecke wird beispielsweise auf Grundlage des Anteils der Hindernisstrecke ermittelt, den der Anwender bereits bewältigt hat, und als Belastungswert bereitgestellt. Beispielsweise hat der Anwender die Hälfte einer vorgegebenen zu verrichtenden Arbeit in der Übungsstrecke erbracht, sofern er die Hälfte der Hindernisstrecke bewältigt hat. Ferner ist denkbar, dass der Belastungswert auf Grundlage der Zeit, in der sich der Anwender in der Hindernisstrecke befindet, und/oder auf Grundlage von physiologischen Messungen mit einer geeigneten Messvorrichtung am Anwender ermittelbar ist.

Die Steuereinheit umfasst ein Kommunikationsmodul und ist beispielsweise als Computer, als mobiles Endgerät, als Mikroprozessor oder als sonstige Recheneinheit, die geeignet ist, Daten zu verarbeiten, ausgebildet. Das Kommunikationsmodul ist zur drahtlosen und/oder kabelgebundenen Datenübertragung ausgebildet Bevorzugt ist das Kommunikationsmodul in die Steuereinheit integriert. Ferner bevorzugt sind die Steuereinheit und das Kommunikationsmodul, zwei separate Geräte, die zum Datenaustausch miteinander verbunden sind, wobei das Kommunikationsmodul beispielsweise einen Hub umfasst. Der Hub ist vorzugsweise zur drahtlosen und/oder zur kabelgebundenen Datenübertragung ausgebildet.

Das wenigstens eine Übungsgerät ist eingerichtet, den Belastungswert an das Kommunikationsmodul der Steuereinheit zu übertragen. Dabei erfolgt die Datenübertragung vorzugsweise kabelgebunden und/oder drahtlos. Vorzugsweise ist das wenigstens eine Übungsgerät ausgebildet, den aktuellen Belastungswert kontinuierlich, beispielsweise sekündlich, zu erfassen und an die Steuereinheit zu übertragen. Insbesondere gibt der Belastungswert an, wie viel Arbeit der Anwender seit Beginn der physischen Belastung an dem wenigstens einen Übungsgerät verrichtet hat.

Das Kommunikationsmodul ist eingerichtet, zusätzlich zum Belastungswert den Atemluftentnahmewert zu empfangen. Die Datenübertragung erfolgt dabei kabelgebunden und/oder bevorzugt drahtlos, beispielsweise über Funk, insbesondere im Megahertz-Bereich, über Bluetooth, WiFi, LoRa oder Zigbee.

Dabei umfasst der Atemluftentnahmewert eine Information über einen aktuellen Atemluftvorrat des Atemschutzgerätes, einen aktuellen Atemluftverbrauch pro Zeiteinheit und/oder einen aktuellen Atemluftverbrauch seit Beginn der Nutzung des Atemschutzgerätes oder seit Beginn der physischen Belastung des Anwenders an dem wenigstens einen Übungsgerät. Der Atemluftvorrat und der Atemluftverbrauch umfassen bevorzugt einen Volumenwert. Vorzugsweise ist das Kommunikationsmodul ferner eingerichtet, kontinuierlich, beispielsweise sekündlich, einen aktuellen Atemluftentnahmewert zu empfangen.

Die Steuereinheit ist eingerichtet, auf Grundlage des Atemluftentnahmewertes und des Belastungswertes den Übungsstatus zu ermitteln. Besonders bevorzugt ist die Steuereinheit eingerichtet, den Übungsstatus kontinuierlich, insbesondere während der Durchführung einer Übung des Anwenders in der Übungsanlage oder bei Benutzung des wenigstens einen Übungsgerätes durch den Anwender, zu ermitteln, wobei ein jeweiliger aktueller Übungsstatus auf dem kontinuierlich, beispielsweise sekündlich, erfassten aktuellen Atemluftentnahmewert und dem aktuellen Belastungswert basiert.

Zur Ermittlung des Übungsstatus werden der Atemluftentnahmewert und der Belastungswert ins Verhältnis gesetzt und mit einem Grenzwert verglichen. Wird der Grenzwert überschritten ist der Übungsstatus negativ, andernfalls ist der Übungsstatus positiv. Ein negativer Übungsstatus hat die Bedeutung, dass der Anwender nicht oder unzureichend für die Verwendung eines Atemschutzgerätes ist, beispielsweise, weil er zu viel Atemluft verbraucht, wobei die noch vorhandene Atemluft des Atemschutzgerätes nicht mehr ausreichen wird, um die Übung in der Übungsanlage zu beenden. Ein positiver Übungsstatus hat die Bedeutung, dass der Anwender für einen Einsatz des Atemschutzgerätes geeignet ist, wobei beispielsweise die noch vorhandene Atemluft des Atemschutzgerätes ausreichend ist, dass der Anwender die Übung in der Übungsanlage beenden kann, somit also die zu verrichtende Arbeit aller Übungsgeräte der Übungsanlage bewältigen kann. Weitere Abstufungen des Übungsstatus und/oder mehrere Grenzwerte sind denkbar.

Bevorzugt ist die Steuereinheit eingerichtet, den Übungsstatus auf Grundlage eines vorgegebenen Gesamtatemluftvolumens und eines vorgegebenen Gesamtbelastungswertes zu ermitteln. Das Gesamtatemluftvolumen und der Gesamtbelastungswert sind beispielsweise von einem Übungsleiter vorgegeben und in der Steuereinheit hinterlegt, von der Steuereinheit empfangbar und/oder von der Steuereinheit abrufbar. Das Gesamtatemluftvolumen umfasst jenen Atemluftvolumenwert, der für die zu verrichtende Arbeit des Anwenders an dem wenigstens einen Übungsgerät vorgesehen ist, wobei der Gesamtbelastungswert die zu verrichtende Arbeit festlegt. Der Übungsstatus ergibt sich hierbei aus dem Vergleich des Atemluftentnahmewertes und des Belastungswertes mit dem Gesamtvolumen und dem Gesamtbelastungswert.

Beispielsweise beschreibt die DIN 14093 und die Feuerwehrdienstvorschrift 7, dass der Anwender eines Atemschutzgerätes, in diesem Fall ein Feuerwehrmann mit einem Pressluftatmer, für einen Gesamtbelastungswert von 80 Kilojoule ein maximales Gesamtatemluftvolumen von 1600 Liter verbrauchen darf, um für den Einsatz unter Atemschutz geeignet zu sein. Dementsprechend ergibt sich ein Verhältnis von 20 Liter verbrauchter Atemluft pro 1 Kilojoule verrichteter Arbeit, welches mit dem Verhältnis von dem Atemluftentnahmewert und dem Belastungswert durch die Steuereinheit verglichen wird. Benötigt der Feuerwehrmann weniger als oder genau 20 Liter pro Kilojoule während der Übung in der Übungsanlage, so ist davon auszugehen, dass er die Übung mit der noch vorhandenen Atemluft im Atemluftvorrat vollständig durchführen kann und diese besteht. In diesem Fall ist der Ergebniswert positiv. Benötigt der Anwender mehr als 20 Liter pro Kilojoule, so ist davon auszugehen, dass er die Übung mit der noch vorhandenen Atemluft im Atemluftvorrat nicht vollständig durchführen kann und diese nicht besteht. In diesem Fall ist der Ergebniswert negativ.

Ferner ist denkbar, dass die Ermittlung des Übungsstatus durch die Steuereinheit zusätzlich oder alternativ mittelbar oder unmittelbar auf einem Vergleich des Atemluftentnahmewertes und des Belastungswertes mit zuvor aufgenommenen Daten erfolgt. Dabei umfassen die zuvor aufgenommenen Daten Atemluftentnahmewerte und Belastungswerte sowie Übungsstatus von einer Mehrzahl durchgeführter Übungen in der Übungsanlage. Dabei ist die Steuereinheit eingerichtet, auf Grundlage der aktuellen Werte und der zuvor aufgenommenen Daten einen aktuellen Übungsstatus zu ermitteln.

Die Steuereinheit ist eingerichtet, gemäß des ermittelten Übungsstatus eine Information zur Ausgabe zu erzeugen. Dabei handelt es sich vorzugsweise um ein Ausgabesignal für eine Anzeigeeinheit, welche den Übungsstatus beispielsweise in Form einer farblichen Statusmeldung darstellt, beispielsweise grün für einen positiven Übungsstatus und rot für einen negativen Übungsstatus. Ferner ist denkbar, dass die Information zusätzlich den Atemluftentnahmewert und/oder das Verhältnis von Atemluftentnahmewert und Belastungswert umfasst und dieser farblich entsprechend dem ermittelten Übungsstatus auf der Anzeigeeinheit dargestellt wird. Die Anzeigeeinheit kann ein Display, eine LED oder eine andere Einheit zur optischen Anzeige sein. Ferner ist denkbar, dass die Information in Form einer elektronischen Nachricht beispielsweise an eine zentrale Überwachungsstelle, einen Computer oder ein mobiles Endgerät übertragbar ist.

Auf vorteilhafte Weise ermöglicht die erfindungsgemäße Übungsanlage eine zuverlässige Prüfung der Eignung eines Anwenders für die Nutzung eines Atemschutzgerätes, wobei die Bewertung auf Grundlage der physischen Belastung und des Atemluftverbrauchs des Anwenders basiert. Ferner ist es möglich, den Übungsstatus kontinuierlich, beispielsweise sekündlich, zu ermitteln, wobei die Übungsanlage besonders sicher ist, da auch während der Übung und in Bezug auf die verrichtete und/oder noch zu verrichtende Arbeit ermittelbar ist, ob ein vorhandener Atemluftvorrat des Atemschutzgerätes für eine den Anwender und der weiteren Belastung des Anwenders ausreichend ist. Dabei sind insbesondere gesundheitliche Risiken durch eine unzureichende Zufuhr von Atemluft erkennbar und somit vermeidbar.

Ein weitere besonderer Vorteil besteht darin, dass mit der erfindungsgemäßen Übungsanlage ermittelbar ist, in welcher Situation der Anwender des Atemschutzgerätes zu viel Atemluft verbraucht, beispielsweise am Anfang oder am Ende der physischen Belastung an dem wenigstens einen Übungsgerät.

Daraus ist ableitbar, was der Anwender für eine wiederholte Übung trainieren muss, um beispielsweise seine Fitness zu steigern und während einer solchen physischen Belastung weniger Atemluft zu verbrauchen.

Gemäß einer bevorzugten Ausführungsvariante der Übungsanlage ist das wenigstens eine Übungsgerät eingerichtet, einen Belastungssollwert von der Steuereinheit zu empfangen. Dabei ist der Belastungssollwert beispielsweise von einem Übungsleiter vorgegeben oder kann von einem Übungsleiter vorgegeben werden. Der Belastungssollwert ist der Wert, über dessen Umfang der Anwender Arbeit an dem wenigstens einen Übungsgerät verrichten muss, bis die Übung an dem wenigstens einen Übungsgerät abgeschlossen ist. Im Fall eines einzelnen Übungsgerät entspricht der Belastungssollwert demzufolge dem zuvor beschriebenen Gesamtbelastungswert. Im Fall einer Mehrzahl von Übungsgeräten ist der Belastungssollwert eines Übungsgerätes lediglich ein Anteil des Gesamtbelastungswertes und für jedes der Mehrzahl von Übungsgeräten individuell vorgebbar.

Auf vorteilhafte Weise lässt sich die zu verrichtende Arbeit, die ein Atemschutzgeräteträger in der Übungsanlagen zu verrichten hat, flexibel an die Bedürfnisse der Übung anpassen und im Fall einer Mehrzahl von Übungsgeräten bedarfsgerecht verteilen.

In einer bevorzugten Ausführungsvariante der Übungsanlage entspricht der Atemluftentnahmewert der Entnahme eines Atemvolumens pro Zeiteinheit, vorzugsweise in Liter pro Sekunde oder Liter pro Minute, aus dem Atemluftvorrat des Atemschutzgerätes. Vorzugsweise ist die Steuereinheit eingerichtet eine Information mit diesem Atemluftentnahmewert zur Ausgabe zu erzeugen.

Auf vorteilhafte Weise ist die die Entnahme des Atemvolumens pro Zeiteinheit mit dem Übungsstatus in einem Ausgabesignal verknüpfbar und ebenfalls anzeigbar. Beispielsweise ist dieser Wert farblich markiert anzeigbar, wobei der Wert grün markiert anzeigbar ist, sofern der Übungsstatus positiv ist und wobei der Wert rot markiert anzeigbar ist, sofern der Übungsstatus negativ ist. Von besonderem Vorteil hierbei ist, dass gleichzeitig die Bewertung und die aktuelle Entnahme des Atemvolumens pro Zeiteinheit anzeigbar sind und der Anwender oder ein Übungsleiter Maßnahmen ergreifen kann. Beispielsweise kann der Anwender auf vorteilhafte Weise durch eine Veränderung seiner Atemfrequenz und/oder seiner Atemtiefe die Entnahme des Atemvolumens pro Zeiteinheit verringern, wobei sich bestenfalls ein negativer Übungsstatus in einen positiven Übungsstatus ändert.

Gemäß einer bevorzugten Ausführungsvariante umfasst die Übungsanlage eine Anzeigeeinheit, die zum Datenaustausch mit der Steuereinheit verbunden ist, wobei die Information über den Übungsstatus auf der Anzeigeeinheit anzeigbar ist. Die Anzeigeeinheit umfasst vorzugsweise einen Bildschirm, eine Segmentanzeige und/oder eine optische Signaleinheit beispielsweise in Form einer LED.

Auf vorteilhafte Weise ist der Übungsstatus auf der Anzeigeeinheit anzeigbar, so dass der Anwender des Atemschutzgerätes und/oder ein Übungsleiter, der die Übung und die Übungsanlage betreut, auf den angezeigten Übungsstatus reagieren kann.

Ferner betrifft die Erfindung ein Übungssystem mit einem Atemschutzgerät zur Versorgung eines Anwenders mit Atemluft und mit einer erfindungsgemäßen Übungsanlage oder mit einer Übungsanlage nach einer der vorherigen Ausführungsvarianten. Dabei umfasst das Atemschutzgerät eine Messeinheit zur Ermittlung des Atemluftentnahmewertes und eine Kommunikationseinheit zur Übertragung des Atemluftentnahmewertes. Bei der Kommunikationseinheit handelt es sich vorzugsweise um ein Funkmodul zur Datenübertragung über Funk, insbesondere im Megahertz-Bereich, über Bluetooth, WiFi, LoRa oder Zigbee.

Die Messeinheit ist eingerichtet, den Atemluftentnahmewert zu ermitteln, der vorzugsweise einen aktuellen Verbrauch von Luft aus einem Atemluftvorrat des Atemschutzgerätes, einen aktuellen Restfüllstand des Atemluftvorrates, einen Druckwert einer Gasflasche des Atemschutzgerätes, einen Atemluftentnahmewert pro Zeiteinheit und/oder eine Restlaufzeit bis zum Verbrauch eines gesamten Atemluftvorrates des Atemschutzgerätes umfasst.

Wie zuvor beschrieben ist die Steuereinheit der Übungsanlage zumindest eingerichtet, auf Grundlage des Atemluftentnahmewertes und des Belastungswertes den Übungsstatus zu ermitteln und eine Information über den Übungsstatus zur Ausgabe zu erzeugen.

Die Erfindung betrifft ferner ein Verfahren zur automatisierten Überwachung eines Übungsstatus eines Anwenders eines Atemschutzgerätes in einer Übungsanlage mit wenigstens einem Übungsgerät zur physischen Belastung des Anwenders. Dabei weist das Verfahren folgende Schritte auf:
- Ermitteln eines Atemluftentnahmewertes, insbesondere durch das Atemschutzgerät,
- Senden des Atemluftentnahmewertes an eine Steuereinheit,
- Ermitteln eines Belastungswertes, insbesondere durch das wenigstens eine Übungsgerät oder einen Sensor, der mit dem Übungsgerät verknüpft ist, wobei der Belastungswert einer verrichteten Arbeit des Anwenders an dem wenigstens einen Übungsgerät entspricht,
- Senden des Belastungswertes an die Steuereinheit,
- Ermitteln des Übungsstatus auf Grundlage des Atemluftentnahmewertes und des Belastungswertes durch die Steuereinheit, und
- Erzeugen eines Ausgabesignals mit einer Information über den Übungsstatus

Zunächst wird der Atemluftentnahmewert ermittelt, wobei es sich um einen aktuellen Verbrauch eines Atemluftvorrates, einen aktuellen Restfüllstand eines Atemluftvorrates, einen Druckwert einer Gasflasche, einen Entnahmewert pro Zeiteinheit und/oder eine Restlaufzeit bis zum Verbrauch eines gesamten Atemluftvorrates des Atemschutzgerätes handelt.

Ferner wird der Belastungswert vorzugsweise ermittelt. Der Belastungswert und der Atemluftentnahmewert werden kabelgebunden oder bevorzugt drahtlos an die Steuereinheit übertragen, welche auf Grundlage dieser beiden Werte den Übungsstatus ermittelt und eine Information über den Übergangsstatus zur Ausgabe erzeugt.

Auf vorteilhafte Weise ermöglicht das erfindungsgemäße Verfahren eine zuverlässige Prüfung der Eignung eines Anwenders für die Nutzung eines Atemschutzgerätes, wobei die Bewertung auf Grundlage der physischen Belastung und des Atemluftverbrauchs des Anwenders basiert. Ferner ist es möglich, den Übungsstatus kontinuierlich, beispielsweise sekündlich, zu ermitteln, wobei das Verfahren besonders sicher ist, da auch während der Übung und in Bezug auf die verrichtete und/oder noch zu verrichtende Arbeit ermittelt wird, ob ein vorhandener Atemluftvorrat des Atemschutzgerätes für den Anwender ausreichend ist. Dabei können insbesondere gesundheitliche Risiken durch unzureichende Zufuhr von Atemluft frühzeitig erkannt und Gesundheitsschäden zuverlässig vermieden werden.

Ferner betrifft die Erfindung ein Computerprogrammprodukt umfassend Befehle, die bei Ausführung durch eine Steuereinheit die Steuereinheit dazu veranlassen, einen Übungsstatus zu ermitteln und ein Ausgabesignal mit einer Information über den Übungsstatus zur Ausgabe zu erzeugen, wobei der Übungsstatus auf Grundlage eines Atemluftentnahmewertes und eines Belastungswertes ermittelt wird. Dabei umfasst die Steuereinheit vorzugsweise einen Computer oder einen Mikroprozessor sowie ein Kommunikationsmodul zum Empfang des Atemluftentnahmewertes und des Belastungswertes.

Weitere Merkmale, Aufgaben und Wirkungen der Erfindung ergeben sich aus der folgenden Beschreibung spezieller Ausführungsbeispiele und den beigefügten Figuren. Es werden Ausführungsbeispiele der Erfindung beschrieben, ohne den allgemeinen Erfindungsgedanken zu beschränken.

In den Figuren zeigt:
- Fig. 1:: eine schematische Darstellung einer Übungsanlage sowie eines Atemschutzgerät und einer Anzeigeeinheit,
- Fig. 2:: eine schematische Darstellung eines Übungssystems, und
- Fig. 3:: ein Ablaufdiagramm einer Ausführungsform des erfindungsgemäßen Verfahrens.

Nachstehend werden Ausführungsbeispiele der Erfindung anhand der beigefügten Figuren im Einzelnen beschrieben. Dabei sind gleichartige Bauteile in mehreren Figuren jeweils mit den gleichen Bezugszeichen versehen.

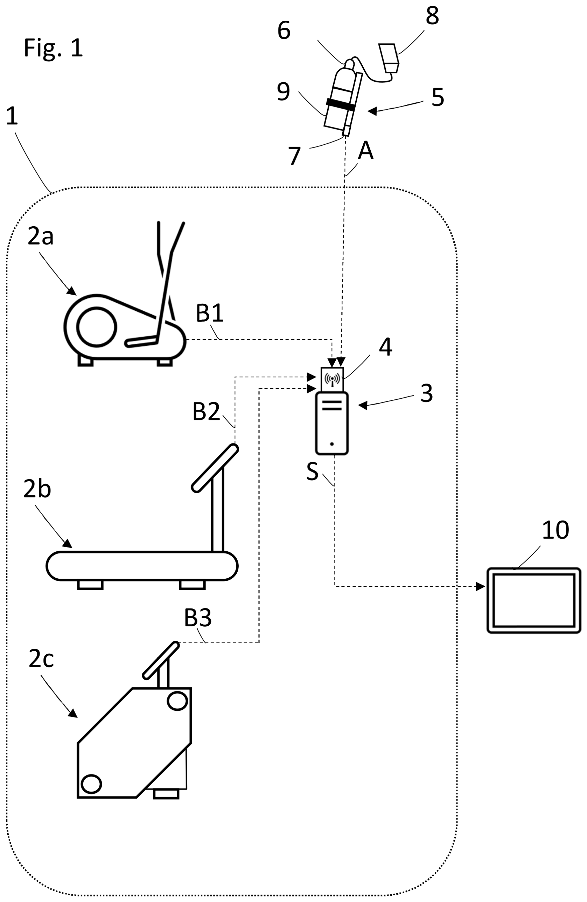

Fig. 1 zeigt eine schematische Darstellung einer Übungsanlage 1 sowie einem Atemschutzgerät 5 und einer Anzeigeeinheit 10. Die Übungsanlage 1 umfasst eine Steuereinheit 3 mit einem Kommunikationsmodul 4. Bei der Steuereinheit 3 handelt es sich um einen Computer mit einem Bluetooth-Modul als Kommunikationsmodul 4. Über das Kommunikationsmodul 4 ist die Steuereinheit 3 zum Datenaustausch mit drei Übungsgeräten 2a, 2b, 2c verbunden, die ebenfalls von der Übungsanlage 1 umfasst sind. Die drei Übungsgeräte 2a, 2b, 2c umfassen ein erstes Übungsgerät 2a ausgebildet als Crosstrainers, ein zweites Übungsgerät 2b ausgebildet als Laufband und ein drittes Übungsgerät 2c ausgebildet als Endlosleiter. Die drei Übungsgeräte 2a, 2b, 2c sind beispielhaft gewählt. Denkbar sind andere Kombinationen von Übungsgeräten sowie weniger oder mehr Übungsgeräte. Die Steuereinheit 3 ist ferner zum Datenaustausch mit einem nicht von der Übungsanlage umfassten Atemschutzgerät 5 und einer nicht von der Übungsanlage umfassten Anzeigeeinheit 10 verbunden. Das Atemschutzgerät umfasst eine Messeinheit 6 zur Ermittlung eines Atemluftentnahmewertes A, einen Atemluftvorrat 9 ausgebildet als Atemgasflasche, eine Atemschutzmaske 8, die mit dem Atemluftvorrat 9 fluidkommunizierend verbunden ist, und eine Kommunikationseinheit 7 ausgebildet als Bluetooth-Modul. Das Atemschutzgerät 5 ist per Bluetooth über das Kommunikationsmodul 4 mit der Steuereinheit 3 verbunden und die Anzeigeeinheit 10 ist per Datenkabel mit der Steuereinheit 3 verbunden. Denkbar ist auch, dass die Anzeigeeinheit 10 ebenfalls über das Kommunikationsmodul 4 mit der Steuereinheit 3 verbunden ist. Ferner ist denkbar, dass die Anzeigeeinheit 10 von der Übungsanlage 1 umfasst ist.

Die Steuereinheit 3 ist eingerichtet, einen ersten Belastungswert B1, einen zweiten Belastungswert B2 und einen dritten Belastungswert B3 von einem jeweiligen Übungsgerät 2a, 2b, 2c sowie einen Atemluftentnahmewert A von einem Atemschutzgerät 5 zu empfangen und zu verarbeiten. Ferner ist die Steuereinheit 3 eingerichtet ein Ausgabesignal S auf Grundlage des Belastungswertes und des Atemluftentnahmewertes zu erzeugen und an die Anzeigeeinheit 10 zu senden. Das Empfangen von Belastungswerten B1, B2, B3 und/oder Atemluftentnahmewerten A kann kontinuierlich, beispielsweise sekündlich, oder ereignisbasiert erfolgen. Ereignisbasiert bedeutet, dass insbesondere zu Zeitpunkten an denen sich die Belastungswerte B1, B2, B3 und/oder der Atemluftentnahmewert A ändert, diese Werte vom jeweiligen Übungsgerät 2a, 2b, 2c und Atemschutzgerät 5 gesendet und von der Steuereinheit 3 empfangen und verarbeitet werden. Dabei ändern sich dieser Werte insbesondere bei Benutzung des jeweiligen Übungsgerätes 2a, 2b, 2c und/oder des Atemschutzgerätes 5.

Das Ausführungsbeispiel gemäß Fig. 1 dient der grundsätzlichen Beschreibung der Übungsanlage 1, wobei die Steuereinheit 3 eingerichtet ist, einen Belastungswert B1, B2, B3 und einen Atemluftentnahmewert A zu empfangen und auf deren Grundlage einen Übungsstatus zu ermitteln sowie ein Ausgabesignal S mit einer Information über den Übungsstatus zu erzeugen. Dabei gibt der Übungsstatus an, ob ein Verhältnis von der verbrauchten Atemluft und der an zumindest einem der Übungsgeräte 2a, 2b, 2c verrichteten Arbeit einen vorgegebenen Grenzwert einhält. Dabei ist die Steuereinheit 3 eingerichtet diesen Vergleich auf Grundlage des Belastungswertes B1, B2, B3 sowie des Atemluftentnahmewertes A und des vorgegebenen Grenzwertes durchzuführen und als Ergebnis ein Ausgabesignal S zu erzeugen, dass eine Information über den Übungsstatus umfasst. Der Übungsstatus ist beispielsweise positiv, sofern der Grenzwert eingehalten wurde, und negativ, sofern der Grenzwert überschritten wurde.

Das erzeugte Ausgangssignal S sendet die Steuereinheit 3 gemäß Fig. 1 über an die Anzeigeeinheit 10, welche die Information über den Übungsstatus anzeigt. In diesem Ausführungsbeispiel ist die Anzeigeeinheit 10 ein Display, wobei die Information über den Übungsstatus beispielsweise als Statussymbol anzeigbar ist. Ferner ist denkbar, dass die Steuereinheit zusätzlich das Verhältnis von Atemluftentnahmewert A und Belastungswert B1, B2, B3 an die Anzeigeeinheit sendet, wobei dieses Verhältnis entsprechend dem ermittelten Übungsstatus farblich markiert anzeigbar ist, beispielsweise grün bei einem positiven Übungsstatus und rot bei einem negativem Übungsstatus.

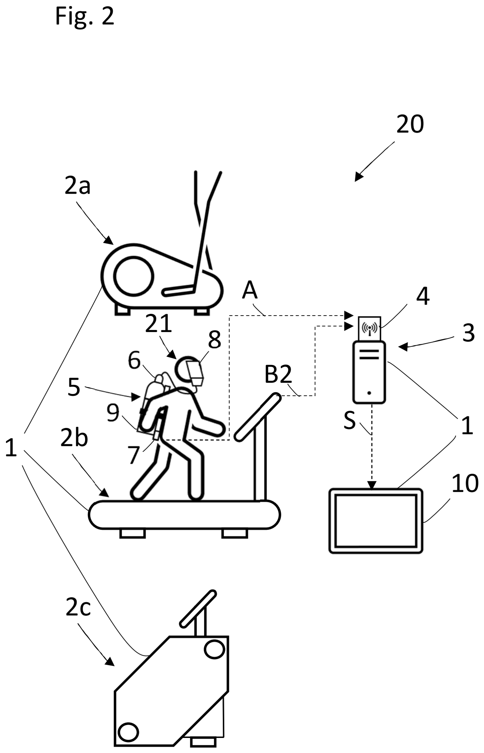

Fig. 2 zeigt eine schematische Darstellung eines Übungssystems 20 umfassend eine Übungsanlage 1 und ein Atemschutzgerät 5. Ferner zeigt Fig. 2 einen Anwender 21, der das Übungssystem 20 zur Feststellung seiner Eignung für das Tragen des Atemschutzgerätes 5 unter Belastungsbedingungen benutzt. Dabei verrichtet der Anwender 21 gemäß Fig. 2 Arbeit an einem zweiten Übungsgerät 2b, das von der Übungsanlage 1 umfasst und als Laufband ausgebildet ist.

Die Übungsanlage 1 des Übungssystems 20 umfasst zwei weitere Übungsgeräte 2a, 2c, die als Crosstrainer und Endlosleiter ausgebildet sind, sowie eine Steuereinheit 3 mit einem Kommunikationsmodul 4 und eine Anzeigeeinheit 10. Bei der in Fig. 2 dargestellten Steuereinheit 3 handelt es sich um einen Computer mit einem Bluetooth-Modul als Kommunikationsmodul 4. Über das Kommunikationsmodul 4 sind Daten zwischen der Steuereinheit 3 den drei Übungsgeräten 2a, 2b, 2c übertragbar. Ferner ist die Steuereinheit 3 mit der Anzeigeeinheit 10, das als Display ausgebildet ist, zum Datenaustausch verbunden.

Das Atemschutzgerät 5 des Übungssystems 20 umfasst eine Messeinheit 6 zur Ermittlung eines Atemluftentnahmewertes A, einen Atemluftvorrat 9, der als Atemgasflasche ausgebildet ist, eine Atemschutzmaske 8, die mit dem Atemluftvorrat 9 fluidkommunizierend verbunden ist, und eine Kommunikationseinheit 7, die als Bluetooth-Modul ausgebildet ist. Das Atemschutzgerät 5 ist zum Datenaustausch per Bluetooth über das Kommunikationsmodul 4 mit der Steuereinheit 3 verbunden.

Die Steuereinheit ist eingerichtet, einen Übungsstatus des in der Übungsanlage 1 trainierenden Anwenders 21 auf Grundlage des zweiten Belastungswertes B2 und des Atemluftentnahmewertes A zu ermitteln. Das zweite Übungsgerät 2b ermittelt bei Benutzung durch den Anwender 21, also während der Anwender 21 Arbeit an dem zweiten Übungsgerät 2b verrichtet, den zweiten Belastungswert B2 und sendet ihn per Bluetooth an das Kommunikationsmodul 4 der Steuereinheit 3. Ferner ermittelt das Atemschutzgerät 5 mit der Messeinheit 6 den Atemluftentnahmewert A während der Benutzung des zweiten Übungsgerätes 2b durch den Anwender 21 und sendet den Atemluftentnahmewert A per Bluetooth an das Kommunikationsmodul 4 der Steuereinheit 3. Die Steuereinheit 3 ist eingerichtet das Verhältnis des empfangenen Atemluftentnahmewertes A und des Belastungswertes B2 zu ermitteln. Dieses Verhältnis gibt also an, wieviel Atemluft der Anwender 21 aus dem Atemluftvorrat 9 pro verrichteter Arbeit am zweiten Übungsgerät 2b verbraucht. Ferner ist die Steuereinheit eingerichtet, das Verhältnis von Atemluftentnahmewert A und Belastungswert B2 mit einem Grenzwert zu vergleichen und den Übungsstatus zu ermitteln. Der Grenzwert ergibt sich dabei aus dem Verhältnis eines vorgegebenen Gesamtatemvolumens und eines Gesamtbelastungswertes. Diese Werte sind von der Steuereinheit und können beispielsweise von einem Übungsleiter festgelegt werden. Das Gesamtvolumen gibt an, wieviel Atemluft der Anwender 21 für den vorgegebenen Gesamtbelastungswert verbrauchen darf. Dabei gibt der Gesamtbelastungswert die verrichtete Arbeit an, die der Anwender 21 insgesamt verrichten soll, um die Übung zu beenden.

Ist das Verhältnis des Atemluftentnahmewertes A und des Belastungswertes B2 größer als der Grenzwert, so ist der Übungsstatus negativ, andernfalls ist der Übungsstatus positiv. Die Steuereinheit erzeugt ein Ausgabesignal S mit einer Information über den Übungsstatus und sendet es an die Anzeigeeinheit, welche diese Information anzeigt. Ein negativer Übungsstatus bedeutet, dass der Anwender 21 zu viel Atemluft für die bisher verrichtete Arbeit verbraucht hat und ggf. nicht geeignet ist ein Atemschutzgerät 5 unter Belastungsbedingungen zu verwenden. Als Folge könnte es notwendig sein, dass der Anwender 21 ein angepassten Training zur Verbesserung seiner Kondition durchführt und die Übung zu einem späteren Zeitpunkt wiederholt. Ferner kann der Anwender versuchen seine Atmung mit geeigneten Atemtechniken während der Übung anzupassen, um die von ihm verbrauchte Atemluft zu verringern, wobei ein weiterer ermittelter Übungsstatus positiv sein kann.

Die Ermittlung des Übungsstatus durch die Steuereinheit 3 kann kontinuierlich, beispielsweise sekündlich, mit den aktuellen Atemluftentnahmewerten A und den aktuellen Belastungswerten B2 erfolgen. Ferner kann die Steuereinheit eingerichtet sein, einen oder mehrere Übungsstatus für die Übung an einem jeweiligen Übungsgerät 2a, 2b, 2c auf den jeweils aktuellen Belastungswerten B1, B2, B3 und den aktuellen Atemluftentnahmewerten A zu ermitteln, wobei der Anwender 21 nacheinander an den verschiedenen Übungsgeräten Arbeit verrichtet.

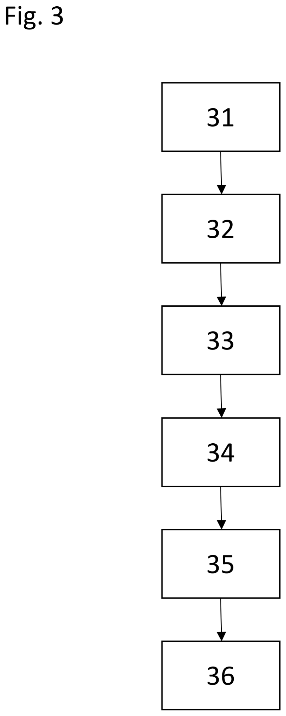

Fig. 3 zeigt schematisch ein Ablaufdiagramm einer bevorzugten Ausführungsform des erfindungsgemäßen Verfahrens. In einem ersten Verfahrensschritt 31 wird ein Atemluftentnahmewert A ermittelt, insbesondere von einer Messeinheit 6 eines Atemschutzgerätes, wobei das Atemschutzgerät von einem Anwender 21 genutzt wird.

Der Atemluftentnahmewert wird in einem zweiten Verfahrensschritt 32 an eine Steuereinheit 3 gesendet. Insbesondere erfolgt das Senden des Atemluftentnahmewertes A von einer Kommunikationseinheit 7 des Atemschutzgerätes 5. Dabei ist die Kommunikationseinheit 7 als Funkeinheit ausgebildet und überträgt den Atemluftentnahmewert A über ein proprietäres Kommunikationsprotokoll und einer Frequenz im Megahertz-Bereich.

In einem dritten Verfahrensschritt 33 wird annähernd zeitgleich, zeitverzögert oder vor der Ausführung eines ersten Verfahrensschrittes 31 ein Belastungswert B1 ermittelt. Der Belastungswert B1 wird von einem Übungsgerät 2a einer Übungsanlage 1 ermittelt und gibt an, wie viel Arbeit der Anwender 21 an dem Übungsgerät 2a verrichtet hat.

Ferner wird der Belastungswert B1 in einem vierten Verfahrensschritt 34 von dem Übungsgerät 2a an die Steuereinheit 3 gesendet. Die Datenübertragung erfolgt dabei kabelgebunden.

In einem fünften Verfahrensschritt 35 ermittelt die Steuereinheit 3 einen Übungsstatus auf Grundlage des Atemluftentnahmewertes A und des Belastungswertes B1. Dabei vergleicht die Steuereinheit 3 das Verhältnis des Atemluftentnahmewertes A zu Belastungswert B1 mit einem vorgegebenen Grenzwert. Der Grenzwert gibt an, wieviel Atemluft pro verrichteter Arbeit der Anwender 21 verbrauchen darf, um die Übung zu bestehen. Im Falle, dass der Grenzwert überschritten ist, ist der Übungsstatus negativ, andernfalls ist der Übungsstatus negativ.

Ferner erzeugt die Steuereinheit 3 ein Ausgabesignal S mit einer Information über den Übungsstatus. Dieses Ausgabesignal S ist geeignet von einer Anzeigeeinheit akustisch und/oder optisch ausgegeben zu werden. Ferner ist das Ausgabesignal alternativ oder zusätzlich geeignet als Nachricht übertragen und angezeigt zu werden.

### Bezugszeichenliste

- 1: Übungsanlage
- 2a: erstes Übungsgerät
- 2b: zweites Übungsgerät
- 2c: drittes Übungsgerät
- 3: Steuereinheit
- 4: Kommunikationsmodul
- 5: Atemschutzgerät
- 6: Messeinheit
- 7: Kommunikationseinheit
- 8: Atemschutzmaske
- 9: Atemgasvorrat
- 10: Anzeigeeinheit
- A: Atemluftentnahmewert
- B1: erster Belastungswert
- B2: zweiter Belastungswert
- B3: dritter Belastungswert
- S: Ausgabesignal
- 20: Übungssystem
- 21: Anwender
- 31: erster Verfahrensschritt
- 32: zweiter Verfahrensschritt
- 33: dritter Verfahrensschritt
- 34: vierte Verfahrensschritt
- 35: fünfter Verfahrensschritt
- 36: sechster Verfahrensschritt

## Patentansprüche

1. Übungsanlage (1) für Anwender (21) eines Atemschutzgerätes (5) umfassend eine Steuereinheit (3) und wenigstens ein Übungsgerät (2a, 2b, 2c) zur physischen Belastung des Anwenders (21), wobei die Steuereinheit (3) ein Kommunikationsmodul (4) umfasst, das eingerichtet ist, einen Atemluftentnahmewert (A), der von einem Atemschutzgerät (5) ermittelbar ist, und einen Belastungswert (B1, B2, B3), der von dem wenigstens einen Übungsgerät (2a, 2b, 2c) ermittelbar ist, zu empfangen, wobei der Belastungswert (B1, B2, B3) eine Information über eine verrichtete Arbeit des Anwenders (21) an dem wenigstens einen Übungsgerät (2a, 2b, 2c) umfasst, und wobei die Steuereinheit (3) eingerichtet ist, auf Grundlage des Atemluftentnahmewertes (A) und des Belastungswertes (B1, B2, B3) einen Übungsstatus zu ermitteln und ein Ausgabesignal (S) zu erzeugen, das eine Information über den Übungsstatus umfasst.

2. Übungsanlage (1) nach Anspruch 1, **dadurch gekennzeichnet, dass** die Steuereinheit (3) eingerichtet ist, den Übungsstatus auf Grundlage eines Gesamtatemluftvolumens und eines Gesamtbelastungswertes zu ermitteln.

3. Übungsanlage (1) nach Anspruch 1 oder 2, **dadurch gekennzeichnet, dass** das wenigstens eine Übungsgerät (2a, 2b, 2c) eingerichtet ist, einen Belastungssollwert von der Steuereinheit (3) zu empfangen.

4. Übungsanlage (1) nach einem der vorherigen Ansprüche, **dadurch gekennzeichnet, dass** der Atemluftentnahmewert (A) einer Entnahme eines Atemluftvolumen pro Zeiteinheit entspricht.

5. Übungsanlage (1) nach einem der vorherigen Ansprüche umfassend eine Anzeigeeinheit (A), **dadurch gekennzeichnet, dass** die Anzeigeeinheit (A) zum Datenaustausch mit der Steuereinheit (1) verbunden ist und die Information über den Übungsstatus auf der Anzeigeeinheit (A) anzeigbar ist.

6. Übungssystem (20) mit einem Atemschutzgerät (5) zur Versorgung eines Anwenders (21) mit Atemluft und einer Übungsanlage (1) nach einem der vorherigen Ansprüche, wobei das Atemschutzgerät (5) eine Messeinheit (6) zur Ermittlung des Atemluftentnahmewertes und eine Kommunikationseinheit (7) zur Übertragung des Atemluftentnahmewertes umfasst.

7. Verfahren zur automatisierten Überwachung eines Übungsstatus eines Anwenders (21) eines Atemschutzgerätes (5) in einer Übungsanlage (1) mit wenigstens einem Übungsgerät (2a, 2b, 2c) zur physischen Belastung des Anwenders (21), aufweisend folgende Schritte:
- Ermitteln eines Atemluftentnahmewertes (A),
- Senden des Atemluftentnahmewertes (A) an eine Steuereinheit (3),
- Ermitteln eines Belastungswertes (B1, B2, B3), wobei der Belastungswert (B1, B2, B3) einer verrichteten Arbeit des Anwenders (21) an dem wenigstens einen Übungsgerät (2a, 2b, 2c) entspricht,
- Senden des Belastungswertes (B1, B2, B3) an die Steuereinheit (3),
- Ermitteln des Übungsstatus auf Grundlage des Atemluftentnahmewertes (A) und des Belastungswertes (B1, B2, B3) durch die Steuereinheit (3), und
- Erzeugen eines Ausgabesignals (S) mit einer Information über den Übungsstatus

8. Computerprogrammprodukt, umfassend Befehle, die bei Ausführung durch eine Steuereinheit (3) die Steuereinheit (1) dazu veranlassen, einen Übungsstatus zu ermitteln und ein Ausgabesignal (S) mit einer Information über den Übungsstatus zu erzeugen, wobei der Übungsstatus auf Grundlage eines Atemluftentnahmewertes (A) und eines Belastungswertes (B1, B2, B3) ermittelt wird.
